# EUROPEAN PATENT SPECIFICATION

(11) **EP 3 278 826 B1**
(45) Date of publication and mention of the grant of the patent: **14.04.2021**
(21) Application number: 16771172.0
(22) Date of filing: 04.02.2016
(51) Int. Cl.: A61M 5/20, A61M 5/24, A61M 5/315, A61M 5/50, A61M 5/178

(54) **LOCKING STRUCTURE FOR INJECTION PEN**
VERRIEGELUNGSSTRUKTUR FÜR INJEKTIONSSTIFT
STUCTURE DE VERROUILLAGE POUR STYLO INJECTEUR

(30) Priority: 01.04.2015 CN 201510149971
(43) Date of publication of application: 07.02.2018
(73) Proprietor: CC Biotechnology Corporation, Tainan City (CN)
(72) Inventor: YEH, Chin-Min, Tainan City (TW)
(74) Representative: Viering, Jentschura & Partner mbB Patent- und Rechtsanwälte
(86) International application number: PCT/CN2016/073529
(87) International publication number: WO 2016/155417

(56) References cited:
- EP-A1- 0 554 995
- EP-A1- 2 452 711
- EP-A2- 2 742 962
- EP-B1- 0 956 873
- WO-A1-99/38554
- WO-A1-2005/018721
- CN-A- 101 829 381
- CN-A- 103 170 038
- CN-A- 103 492 003
- TW-U- M 483 803
- US-A1- 2013 324 925

## Description

### 1. Field of the Invention

The present invention relates to a locking device, especially to a locking device for a syringe that safely controls the injection dose and is laborsaving in operation.

### 2. Description of Related Art

In order to achieve multiple purposes such as dose metering, injection time counting, and repeatable use, the conventional syringes include two kinds: a dose metering syringe and a frequency measuring syringe. The structure of the conventional dose metering syringe mainly includes a driving device assembled by multiple driving components mounted in a barrel and connected with a piston rod. A vial is assembled in the barrel. A needle is mounted on a front end of the barrel and is connected with the vial for injection. When the syringe is in an injecting process, the needle is pierced into the skin or the veins of a human body, and the driving device is driven by pushing the piston rod to inject the medication. The dose of the injection can be controlled by the driving device and the movement of the piston rod to achieve the purpose of dose-metering.

However, the conventional dose metering syringe still has an issue of laboriousness. To solve the laboriousness issue, the conventional dose metering syringe has a structure that comprises a large pitch travel structure and a small pitch travel structure, such that the pushing force provided by a user can be effectively transformed to a rotating force. Accordingly, the operation of the conventional syringe is labor-saving.

In addition, WO 2005/018721, EP 0 554 995, TW M483 803, US 2013/324925, EP 2 742 962, and WO 99/38554 each disclose a conventional syringe, but each conventional syringe does not have a locking device to prevent the syringe from being used repeatedly.

However, the conventional dose metering syringe is not locked and can still be applied to injection repeatedly, so the safety of use of the conventional syringe should be improved.

To overcome the shortcomings of the conventional dose metering syringe, the present invention provides a locking device of a syringe to mitigate or obviate the aforementioned problems.

The main objective of the present invention is to provide a locking device of a syringe to keep the syringe from being used repeatedly:
The syringe has a connecting element, a sleeve, a guiding tube, a screwing tube, an injection rod, a pushing rod, and a locking device. The connecting element has a rear end. The sleeve has a front end connected with the rear end of the connecting element. The guiding tube is mounted in the front end of the sleeve and has an annular connection wall formed on an inner surface of the guiding tube and a central hole defined through the connection wall. The screwing tube is mounted in and is moveable relative to the sleeve and the guiding tube. The injection rod extends into a rear end of the sleeve and a rear end of the screwing tube. The pushing rod is mounted in the screwing tube and has a rod body, a dose controlling segment provided with a thread and mounted in the central hole in the guiding tube and an axial hole defined through the rod body into which the injection rod extends._The locking device is disposed between the guiding tube, the pushing rod and the injection rod of the syringe. The guiding tube has an annular connection wall formed on an inner surface of the guiding tube and a central hole defined through the connection wall. The pushing rod has a dose controlling segment provided with a thread and mounted in the central hole in the guiding tube and an axial hole defined in the pushing rod into which the injection rod extends. The locking device has a locking tube, at least one engaging face, at least one through hole, and at least one resilient hook. The locking tube is formed on and protrudes from the connection wall and is mounted around the central hole. The at least one engaging face is formed on a front segment of the injection rod. The at least one through hole is defined radially in the pushing rod at a position adjacent to the dose controlling segment. The at least one resilient hook is formed on the pushing rod, extends respectively into the at least one through hole, and is selectively pushed by the locking tube to abut respectively against the at least one engaging face to prevent the injection device from being pulled backward and to lock the injection device.

Other objects, advantages and novel features of the invention will become more apparent from the following detailed description when taken in conjunction with the accompanying drawings.

### IN THE DRAWINGS

Fig.1 is an exploded perspective view of a syringe with a locking device in accordance with the present invention;
Fig.2 is another exploded perspective view of the syringe in Fig. 1;
Fig.3 is a cross sectional side view of the syringe in Fig. 1;
Fig.4 is an enlarged cross sectional side view of the syringe in Fig. 1;
Fig.5 is another enlarged cross sectional side view of the syringe in Fig. 1;
Fig.6 is an enlarged cross sectional end view of the syringe along the line 6-6 in Fig. 3;
Fig.7 is another cross sectional end view of the syringe along the line 7-7 in Fig. 3;
Fig. 8 is an enlarged partial perspective view of the syringe in Fig. 1;
Fig. 9 is an enlarged operational partial perspective view of the syringe in Fig. 1;
Fig. 10 is an enlarged exploded perspective view of the locking device in Fig. 1;
Fig. 11 is an enlarged cross sectional side view of locking device in Fig. 10;
Figs. 12 to 15 show operational cross sectional side views of the syringe in Fig, 1; and
Fig. 16 is an enlarged cross sectional side view of the locking device in Fig. 10.

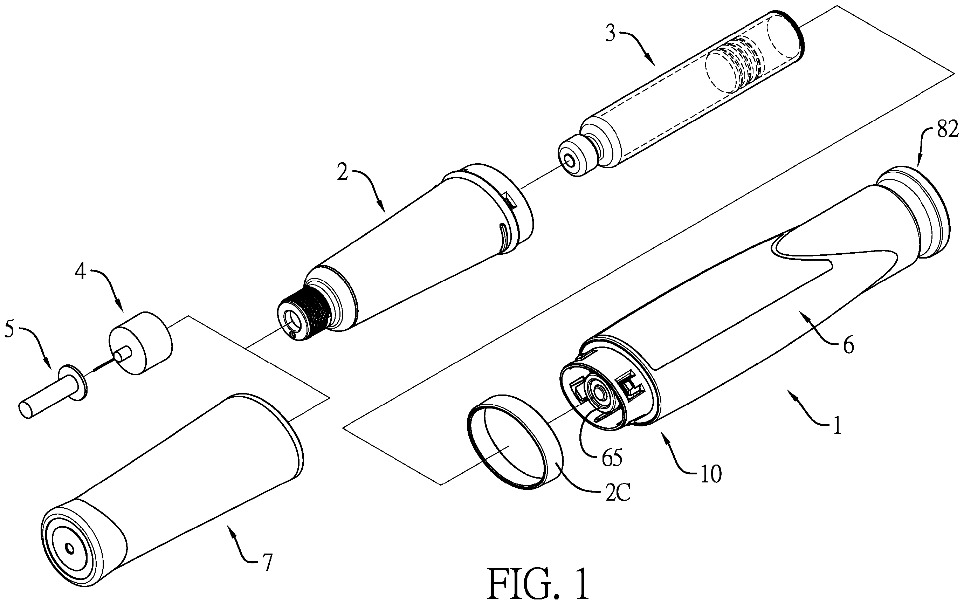

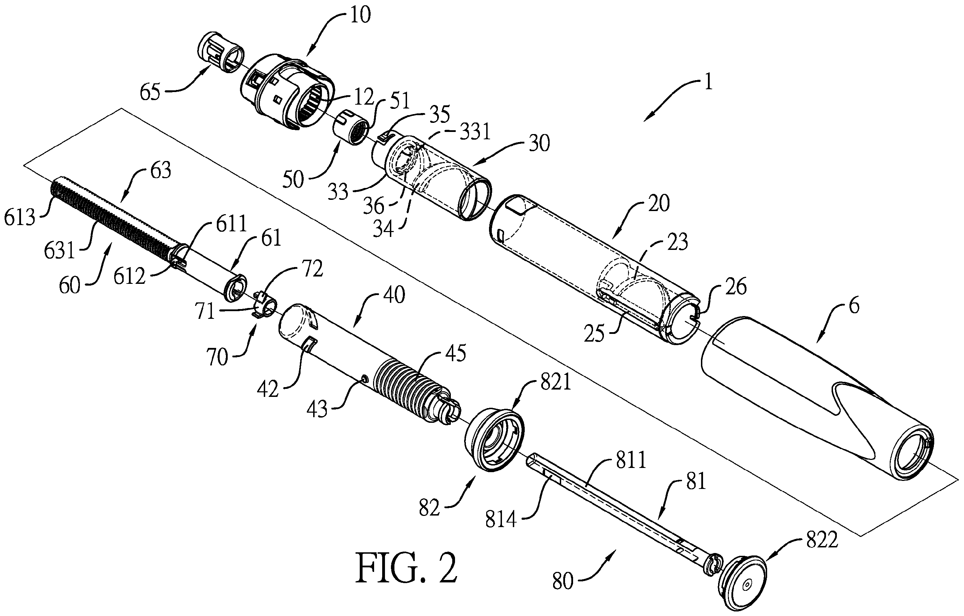

With the reference to Fig. 1, a syringe is shown and has an injection assembly 1 connected with a vial housing 2 for combining with a vial 3, a needle 4 and a needle cap 5. With further reference to Fig. 2, the injection assembly 1 includes a connecting element 10, a sleeve 20, a guiding tube 30, a screwing tube 40, a screwing collar 50, a pushing rod 60, a unidirectional ratchet collar 70, and an injection device 80. In addition, the syringe further comprises a housing 6 and a syringe cap 7.

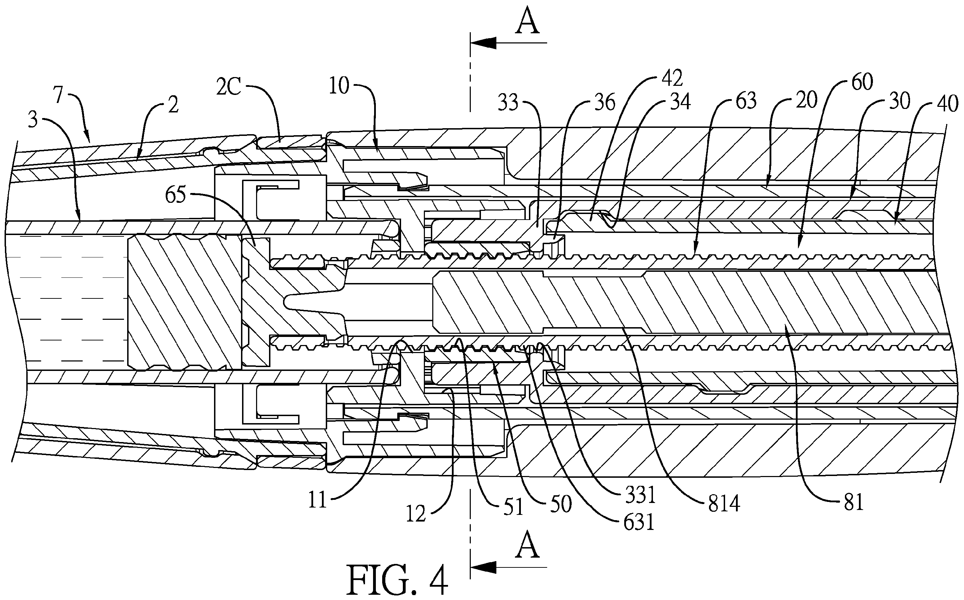

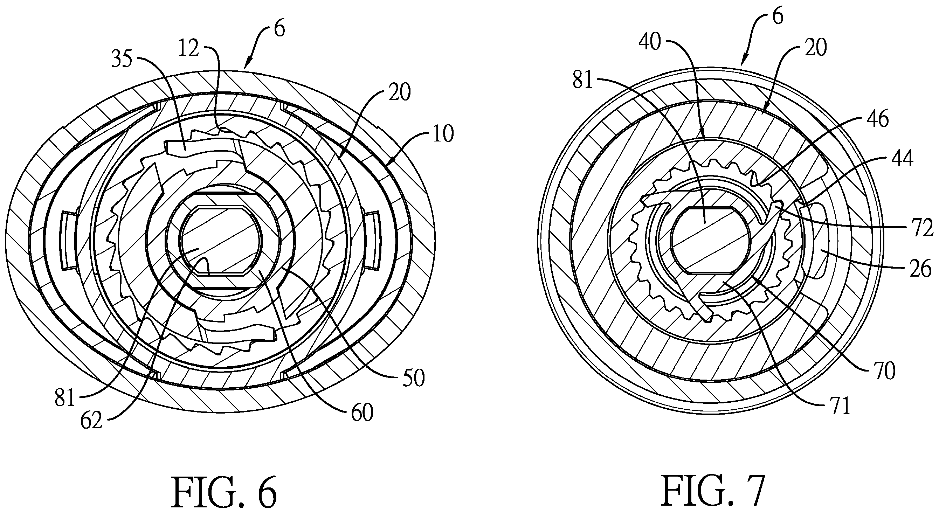

With reference to Figs. 2 to 4, the connecting element 10 comprises a guiding hole 11 and multiple unidirectional teeth 12. The guiding hole 11 is a non-circular hole having two flat faces, such as a polygonal hole or a hole having two flat faces diametrically opposite each other. The sleeve 20 has a front end connected with a rear end of the guiding element 10. The sleeve 20 has a recycling groove defined in an inner surface of the sleeve 20 and composed of a spiral groove 23 and a straight groove 25. The sleeve 20 further has a resilient limiting tab 26 formed on a rear end of the sleeve 20. The guiding tube 30 is mounted in the front end of the sleeve 20 and has a front end extending out of the front end of the sleeve 20 and into the connecting element 10. The guiding tube 30 has an annular connection wall 33, a central hole 331, a spiral guiding groove 34, and a limiting ratchet tab 35. The connection wall 33 is formed on an inner surface of the guiding tube 30. The central hole 331 is defined through the connection wall 33. The guiding groove 34 is defined in the inner surface of the guiding tube 30 and has an end extending to one side of the connection wall 33. The limiting ratchet tab 35 is formed on the front end of the guiding tube 30. With further reference to Fig. 6, the limiting ratchet tab 35 is engaged with the unidirectional teeth 12, such that the guiding tube 30 is limited to be rotated in a unidirectional manner.

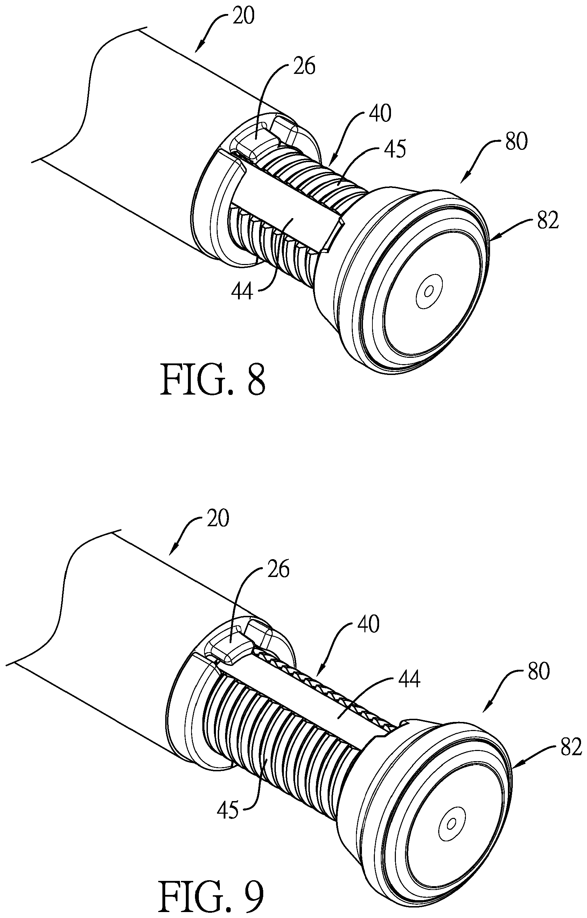

The screwing tube 40 is mounted in and is moveable relative to the guiding tube 30 and the sleeve 20. The screwing tube 40 has a guiding block 42, a guiding protrusion 43, a releasing channel 44, multiple annular grooves 45, and multiple unidirectional ratchet teeth 46. The guiding block 42 is formed on a front end of the screwing tube 40 and is moveably mounted in the guiding groove 34. The guiding protrusion 43 is formed on an outer surface at a middle portion of the screwing tube 40. With reference to Figs. 2 and 8, the releasing channel 44 is longitudinally defined in the outer surface of the screwing tube 40 at the rear end of the screwing tube 40. The annular grooves 45 are defined around the outer surface of the screwing tube 40 and arranged longitudinally at evenly spaced intervals. The guiding protrusion 43 and the releasing channel 44 are diametrically opposite each other. The unidirectional ratchet teeth 46 are formed on the inner surface of the screwing tube 40 at the rear end of the screwing tube 40, and the rear end of the screwing tube 40 extends out of the rear end of the sleeve 20. With reference to Figs. 8 and 9, the guiding protrusion 43 is moveable in the recycling groove composed of the spiral groove 23 and the straight groove 25. With the movement of the guiding protrusion 43 in the spiral groove 23 and the straight groove 25, the limiting tab 26 can be shifted between the releasing channel 44 and the annular grooves 45.

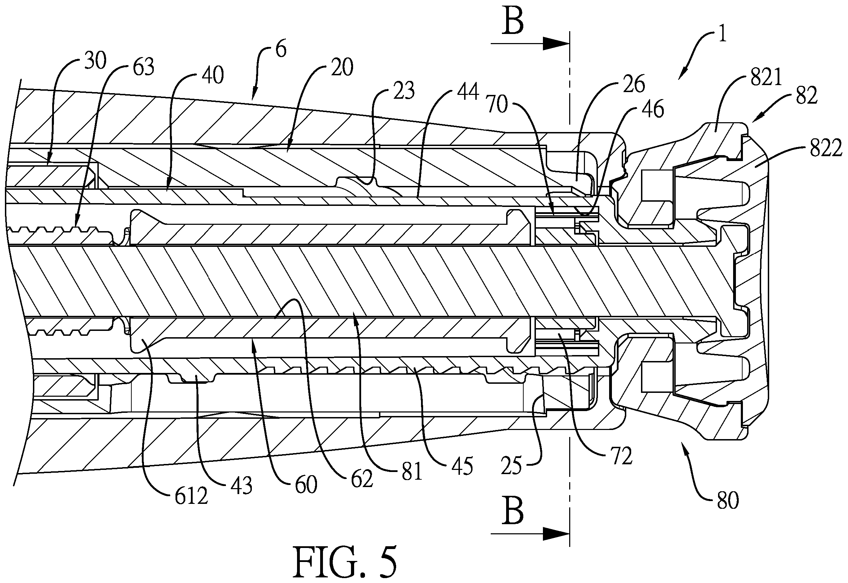

With reference to Figs. 2 to 5, the screwing collar 50 is mounted in the front end of the guiding tube 30. The pushing rod 60 is mounted in the screwing tube 40 and has a rod body 61 and a rod tube 65. The rod body 61 has a non-circular cross section corresponding to the shape of the guiding hole 11 . The rod body 61 has an axial hole 62 defined axially through the rod body 61. The rod body 61 has a dose controlling segment 63 formed on a front segment of the rod body 61 and provided with a thread 631. The thread 631 has a pitch smaller than a pitch of the spiral guiding groove 34. The dose controlling segment 63 extends through the central hole 331 in the connection wall 33, is screwed with a threaded hole 51 in the screwing collar 50, and extends through the non-circular guiding hole 11. Accordingly, the pushing rod 60 can be moved linearly. An engaging hole 613 is defined in the front end of the rod body 61. The rod tube 65 is engaged with the engaging hole 613 to be securely connected with the rod body 61.

The unidirectional ratchet collar 70 has a collar body 71 and multiple unidirectional ratchet tabs 72 formed on and protruding from an outer surface of the collar body 71. With further reference to Fig. 7, the unidirectional ratchet tabs 72 are engaged with the unidirectional ratchet teeth 46.

The injection device 80 comprises an injection rod 81 and an end cap 82 mounted moveably on a rear end of the injection rod 81. The injection rod 81 has a rod body 811 having a non-circular cross section and extending into the rear end of the sleeve 20 and the rear end of the screwing tube 40, through the ratchet collar 70, and into the axial hole 62 in the pushing rod 60. The ratchet collar 70 is mounted securely around the rod body 811 of the injection rod 81. With the non-circular shape of the axial hole 62 and the cross section of the rod body 811 and the injection rod 81, the injection rod can only be moved linearly. The end cap 82 has a cap body 821 and a lid 822. The cap body 821 is mounted on the rear ends of the injection rod and the screwing tube 40 that extend out of the sleeve 20. The lid 822 is combined with the cap body 821, and the screwing tube 40 is rotatable relative to the end cap 82.

With reference to Figs. 1 to 3, the vial housing 2 is applied to connect the vial 3 with a front end of the injection assembly 1. The rod tube 65 on the front end of the pushing rod abuts a piston in the vial 3. The needle 4 extends through the front end of the vial housing 2 and into the vial 3. The housing 6 is mounted on the rear end of the connecting element 10 and is mounted around the sleeve 20. The rear ends of the screwing tube 40 and the injection rod 81 extend out of the rear end of the housing 6 and are combined with the end cap 82. The syringe cap 7 is mounted around the vial housing 2.

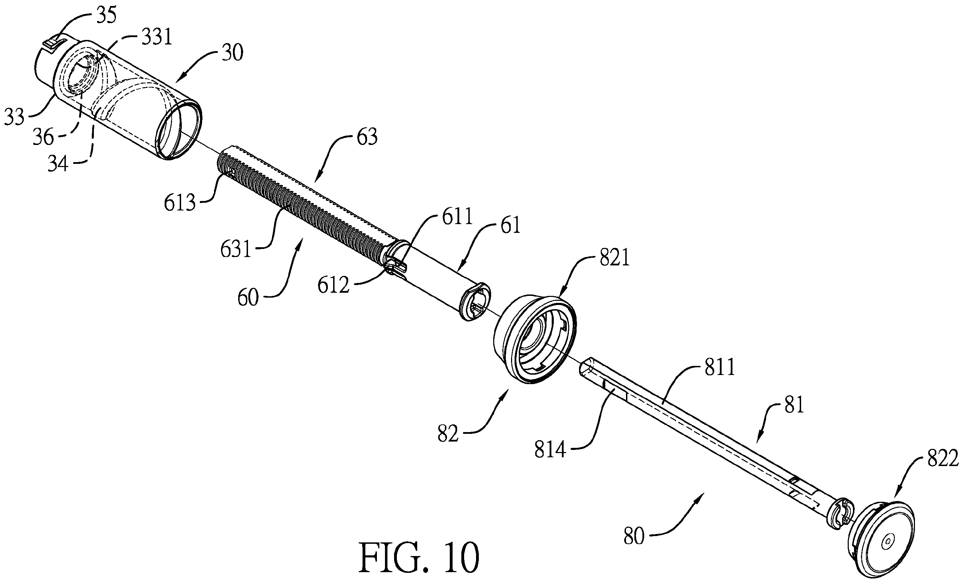

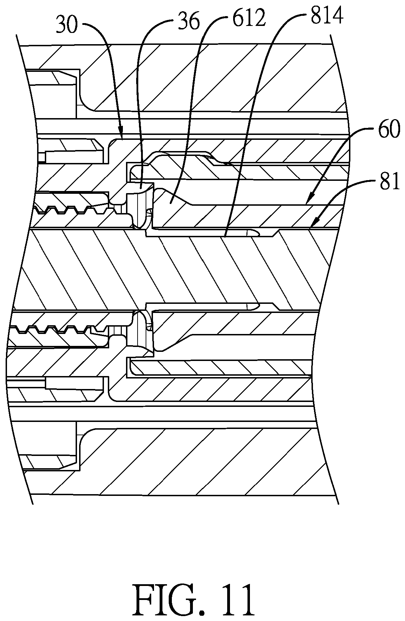

With reference to Figs. 10 and 11, the locking device in accordance with the present invention comprises a locking tube 36, at least one engaging face 814, at least one through hole 611, and at least one resilient hook 612.

The locking tube 36 is formed on and protrudes from a rear side of the connection wall 33 and is mounted around the central hole 331.

The at least one engaging face 814 is formed on a front segment of the rod body 811 of the injection rod 81. Preferably, two engaging faces 814 are implemented and are diametrically opposite to each other.

The at least one through hole 611 is defined radially in the pushing rod 60 at a position adjacent to the dose controlling segment 63. The at least one resilient hook 612 is formed on the pushing rod 60, extend respectively into the at least one through hole 611, and is selectively pushed by the locking tube 36 to abut respectively against the at least one engaging face 814. Preferably, two resilient hooks 612 are implemented and are diametrically opposite each other. The resilient hooks 612 correspond respectively to the travel paths of the engaging faces 814 on the injection rod 81.

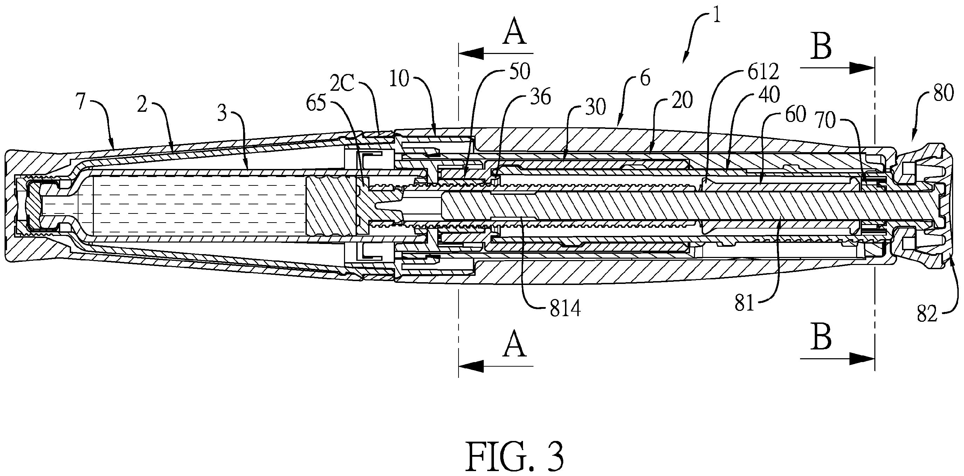

With reference to Fig. 3, when the syringe is in use, a vial 3 is mounted on the front end of the connecting element 10. The rod tube 65 on the front end of the pushing rod 60 extends into the vial 3 and abuts a piston. The vial housing 2 is then mounted around the vial 3 and is connected with the front end of the connecting element 10 via a securing collar 2C. Before the needle 4 is mounted on the syringe, the syringe cap 7 is mounted around the vial housing 2.

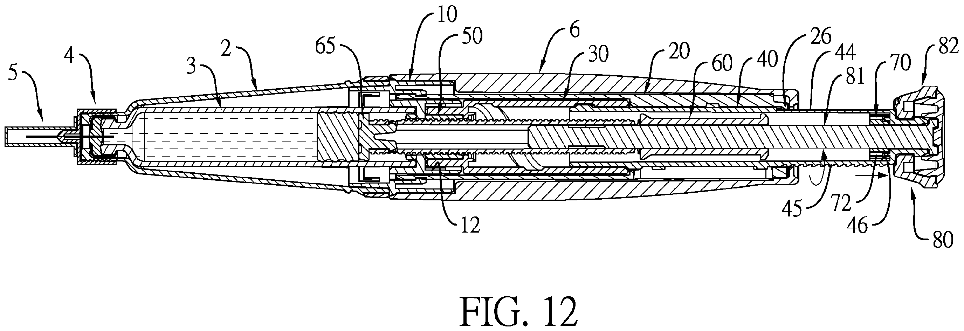

With reference to Figs. 3 and 12, to prepare the injection process, the syringe cap 7 is detached from the syringe and the needle 4 is attached to the front end of the vial housing 2 and extends into the vial 3. The needle cap 5 is mounted around the needle 4 to keep anyone from being stabbed by the needle 4.

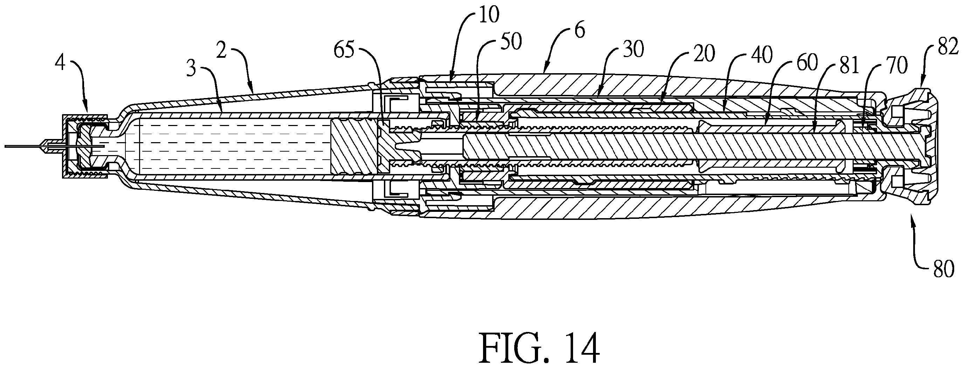

With reference to Figs. 3 and 14, for injection, the injection device 80 is pulled backward to a preparation position. When the injection device 80 is pulled backward, the screwing tube 40 will be moved backward at the same time. At this time, the screwing tube 40 is freely rotating relative to the sleeve 20 and the guiding tube 30, and the unidirectional ratchet tabs 72 on the ratchet collar 70 are engaged with the unidirectional ratchet teeth 46. The limiting ratchet tab 35 on the guiding tube 30 is engaged with the unidirectional teeth 12 in the connecting element 10. Consequently, the rotation direction of the injection rod 81 and the screwing tube 40 is limited, such that the screwing tube 40 can only be rotated in a unidirectional manner. With reference to Fig. 8, at this time, the liming tab 26 on the sleeve 20 moves over the annular groove 45 to generate sounds. With the engagement between the limiting tab 26 with one of the annular grooves 45, the injection device 80 can be prevented from being unintentionally pushed forward. Accordingly, the injection device 80 will not be pushed forward during the backward movement until the screwing tube 40 rotates a full circle. With reference to Fig. 9, when the limiting tab 26 moves into the releasing channel 44, the limiting tab 26 is unlocked.

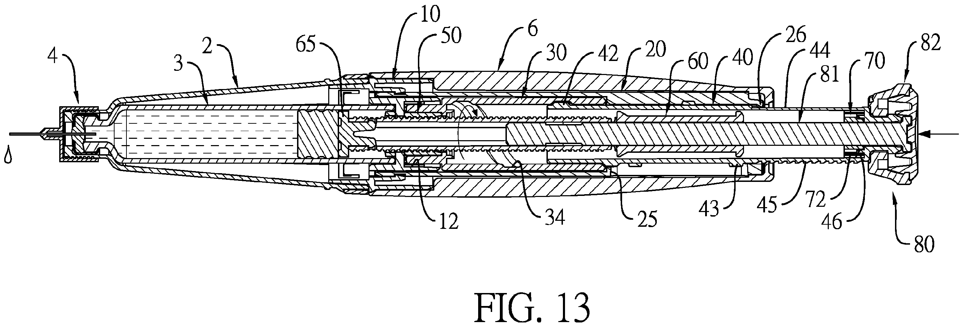

With reference to Figs. 13 and 14, for injection, the needle cap 5 is detached from the syringe to pierce the needle into a human body. The injection device is pushed to inject medication into the human body. The injection device 80 is moved linearly when the end cap 82 is pushed forward, and the screwing tube 40 is simultaneously moved forward. During the movement, because the unidirectional ratchet tabs 72 on the ratchet collar 70 are engaged with the unidirectional ratchet teeth 46 in the screwing tube 40, the rotation direction of the injection rod 81 and the screwing tube 40 is limited. The pushing rod 60 is kept from rotating due to the non-circular guiding hole 11 in the connecting element 10. With further reference to Fig. 9, at this time, the limiting tab 26 is held in the releasing channel 44, and the guiding protrusion 43 is held in the straight groove 25. Thus, the screwing tube 40 can only be moved forward with the injection device 80. With the engagement between the guiding block 42 and the spiral guiding groove 34 in the guiding tube 30, the guiding tube 30 will be driven to rotate. After the screwing tube 40 is moved forward, the screwing tube 40 will not be moved backward again because of the arrangement of the spiral grove 23 and the guiding protrusion 43 of the screwing tube 40. When the guiding block 42 on the screwing tube 40 is rotated for a full circle along the spiral guiding groove 34, the guiding tube 30 is also rotated for a full circle and the screwing collar 50 is also driven to rotate for a full circle. Consequently, the pushing rod 60 is driven to move forward for a predetermined distance to push the piston in the vial and to inject a predetermined dose of medication into the human body. Accordingly, the syringe has a dose metering function. At this time, because the pitch of the spiral guiding groove 34 is larger than that of the thread 631 on the pushing rod 60, the pushing force applied to the injection device 80 can be transformed to a rotating force. Accordingly, the rotating force along the large pitch along the spiral guiding groove 34 can be effectively transmitted to the small pitch along the thread 631, and the medication in the vial can be injected into human bodies completely.

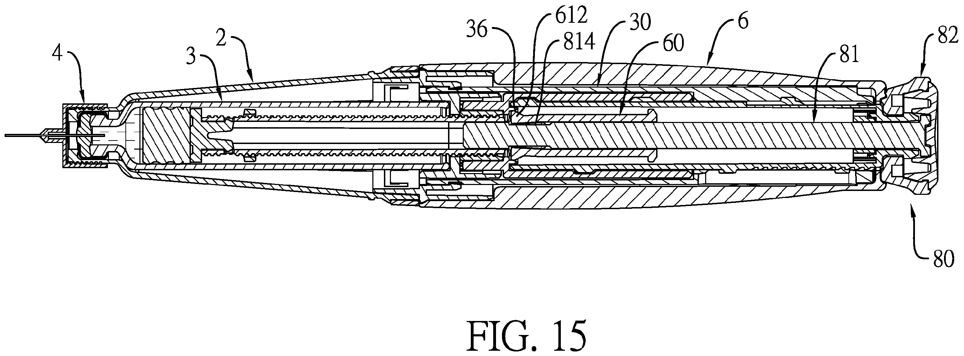

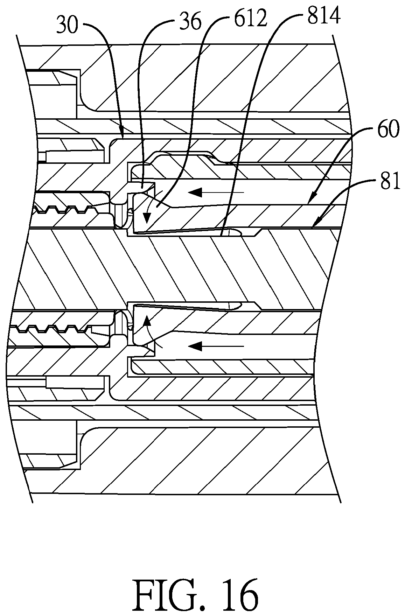

When the medication in the vial 3 is completely injected, with reference to Figs. 15 and 16, the used syringe has to be discarded. When the pushing rod 6 is moved to a position where the resilient hooks 612 are pushed by the locking tube 36 in the guiding tube 30, the resilient hooks 612 will be bent and extend into the pushing rod 60 and will abut against the engaging faces 814 on the injection rod 81. With the engagement between the resilient hooks 612 and the engaging faces 814, the injection device 80 can be prevented from being pulled backward and is locked. Accordingly, the syringe can be kept from being repeatedly used and the safety of use of the syringe can be improved.

Even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A syringe comprising:
a connecting element (10) having a rear end;
a sleeve (20) having a front end connected with the rear end of the connecting element (10);
a guiding tube (30) mounted in the front end of the sleeve (20) and having an annular connection wall (33) formed on an inner surface of the guiding tube (30) and a central hole (331) defined through the connection wall (33);
a screwing tube (40) mounted in and being moveable relative to the sleeve (20) and the guiding tube (30);
an injection rod (81) extending into a rear end of the sleeve (20) and a rear end of the screwing tube (40);
a pushing rod (60) mounted in the screwing tube (40) and having a rod body (61) comprising a dose controlling segment (63) provided with a thread (631), formed on a front segment of the rod body (61), and mounted in the central hole (331) in the guiding tube (30) and an axial hole (62) defined through the rod body (61) into which the injection rod (81) extends, **characterized in that** a locking device is disposed between the guiding tube (30), the pushing rod (60) and the injection rod (81) and comprises
a locking tube (36) formed on and protruding from the connection wall (33) and mounted around the central hole (331);
at least one engaging face (814) formed on a front segment of the injection rod (81);
at least one through hole (611) defined radially in the pushing rod (60) at a position adjacent to the dose controlling segment (63); and
at least one resilient hook (612) formed on the pushing rod (60), extending respectively into the at least one through hole (611), and selectively pushed by the locking tube (36) to abut respectively against the at least one engaging face (814) to prevent the injection rod (81) from being pulled backward and to lock the injection rod (81).

2. The syringe as claimed in claim 1, wherein
two engaging faces (814) are implemented and are diametrically opposite to each other; and
two resilient hooks (612) are implemented and are diametrically opposite each other.

## Patentansprüche

1. Spritze, aufweisend:
eine Verbindungskomponente (10) mit einem hinteren Ende;
eine Hülse (20) mit einem vorderen Ende, das mit dem hinteren Ende der Verbindungskomponente (10) verbunden ist;
ein Führungsrohr (30), das in dem vorderen Ende der Hülse (20) angebracht ist und eine ringförmige Verbindungswand (33), die an einer Innenfläche des Führungsrohrs (30) ausgebildet ist, und ein durch die Verbindungswand (33) definiertes zentrales Loch (331) aufweist;
ein Schraubrohr (40), das in der Hülse (20) und dem Führungsrohr (30) angebracht ist und relativ zu diesen beweglich ist;
eine Injektionsstange (81), die sich in ein hinteres Ende der Hülse (20) und ein hinteres Ende des Schraubrohrs (40) erstreckt;
eine Schubstange (60), die in dem Schraubrohr (40) angebracht ist und einen Stangenkörper (61) aufweist, wobei der Stangenkörper (61) ein Dosissteuerungssegment (63), das ein Gewinde (631) aufweist, das an einem vorderen Abschnitt des Stangenkörpers (61) ausgebildet ist und in dem zentralen Loch (331) in dem Führungsrohr (30) angebracht ist, und ein axiales Loch (62), das durch den Stangenkörper (61) definiert ist, in das sich die Injektionsstange (81) erstreckt, aufweist,
**dadurch gekennzeichnet, dass**
eine Verriegelungsvorrichtung zwischen dem Führungsrohr (30), der Schubstange (60) und der Injektionsstange (81) angeordnet ist und die Verriegelungsvorrichtung aufweist:
ein Verriegelungsrohr (36), das an der Verbindungswand (33) ausgebildet ist und aus dieser herausragt und um das zentrale Loch (331) herum angebracht ist; mindestens eine Eingriffsfläche (814), die an einem vorderen Abschnitt der Injektionsstange (81) ausgebildet ist;
mindestens ein Durchgangsloch (611), das radial in der Schubstange (60) an einer Position neben dem Dosissteuerungssegment (63) definiert ist; und
mindestens einen federnden Haken (612), der an der Schubstange (60) ausgebildet ist und sich in das mindestens eine Durchgangsloch (611) erstreckt und selektiv von dem Verriegelungsrohr (36) gedrückt wird, um an die mindestens eine Eingriffsfläche (814) zu stoßen, um zu verhindern, dass die Injektionsstange (81) nach hinten gezogen wird, und um die Injektionsstange (81) zu verriegeln.

2. Spritze gemäß Anspruch 1, wobei
zwei Eingriffsflächen (814) implementiert sind und einander diametral gegenüberliegen; und
zwei federnde Haken (612) implementiert sind und sich diametral gegenüberliegen.

## Revendications

1. Seringue comprenant :
un élément de liaison (10) ayant une extrémité arrière ;
un manchon (20) ayant une extrémité avant reliée à l'extrémité arrière de l'élément de liaison (10) ;
un tube de guidage (30) monté dans l'extrémité avant du manchon (20) et ayant une paroi de liaison annulaire (33) formée sur une surface interne du tube de guidage (30) et un trou central (331) défini à travers la paroi de liaison (33) ;
un tube de vissage (40) monté dans le manchon (20) et étant mobile par rapport à celui-ci et au tube de guidage (30) ;
une tige d'injection (81) s'étendant dans une extrémité arrière du manchon (20) et une extrémité arrière du tube de vissage (40) ;
une tige de poussée (60) montée dans le tube de vissage (40) et ayant un corps de tige (61) comprenant une section de commande de dose (63) pourvue d'un filet (631), formée sur une section avant du corps de tige (61), et montée dans le trou central (331) dans le tube de guidage (30) et un trou axial (62) défini à travers le corps de tige (61) dans lequel la tige d'injection (81) s'étend, **caractérisé en ce qu'**un dispositif de verrouillage est disposé entre le tube de guidage (30), la tige de poussée (60) et la tige d'injection (81) et comprend
un tube de verrouillage (36) formé sur la paroi de liaison (33) et faisant saillie depuis celle-ci et monté autour du trou central (331) ;
au moins une face de mise en prise (814) formée sur une section avant de la tige d'injection (81) ;
au moins un trou traversant (611) défini radialement dans la tige de poussée (60) à une position adjacente à la section de commande de dose (63) ; et
au moins un crochet élastique (612) formé sur la tige de poussée (60), s'étendant respectivement dans l'au moins un trou traversant (611), et poussé sélectivement par le tube de verrouillage (36) pour venir en butée respectivement contre l'au moins une face de mise en prise (814) pour empêcher la tige d'injection (81) d'être tirée vers l'arrière et pour verrouiller la tige d'injection (81).

2. Seringue selon la revendication 1, dans laquelle
deux faces de mise en prise (814) sont mises en œuvre et sont diamétralement opposées l'une à l'autre ; et
deux crochets élastiques (612) sont mis en œuvre et sont diamétralement opposés l'un à l'autre.
